# EUROPEAN PATENT SPECIFICATION

(11) **EP 3 161 051 B1**
(45) Date of publication and mention of the grant of the patent: **07.07.2021**
(21) Application number: 15723843.7
(22) Date of filing: 20.05.2015
(51) Int. Cl.: C08J 9/00, C07D 493/04, C08J 9/14, C08K 5/00, C08K 5/15

(54) **COMPOSITION FOR PREPARING AN AMORPHOUS POLYMERIC FOAM USING A BENZYLIDENE SORBITOL NUCLEATING AGENT**
ZUSAMMENSETZUNG ZUR HERSTELLUNG EINES AMORPHEN POLYMERSCHAUMS MIT EINEM BENZYLIDENSORBITOL-NUKLEIERUNGSMITTEL
COMPOSITION DE PRÉPARATION D'UNE MOUSSE POLYMÈRE AMORPHE À L'AIDE D'UN AGENT DE NUCLÉATION DU SORBITOL DE BENZYLIDÈNE

(30) Priority: 26.06.2014 US 201414316519
(43) Date of publication of application: 03.05.2017
(73) Proprietor: Clariant Plastics & Coatings Ltd, 4132 Muttenz (CH)
(72) Inventor: SCHOLZ, Patricia, 22049 Hamburg (DE); WEGNER, Jan-Erik, 22041 Hamburg (DE); NEUBER, Frank William, Boyce, Virginia 22620 (US)
(74) Representative: Appleyard Lees IP LLP
(86) International application number: PCT/EP2015/001026
(87) International publication number: WO 2015/197152

(56) References cited:
- CN-A- 102 816 363
- JP-A- H02 284 934
- JP-A- 2004 269 769
- JP-A- 2009 242 808
- None

## Description

The present invention relates to compositions for the preparation of a polymeric foam with improved thermal properties, and to a foamed polymeric article obtainable therefrom, and to a method for preparing such a polymeric foam or article.

In response to environmental concerns, there has been an evolution from using hydrochlorofluorocarbon (HCFC) foam blowing agents to using carbon dioxide and/or hydrocarbons and alcohols. Unfortunately, as a result of this change, the thermal conductivity of foam materials has increased due to the higher conductivity of these new blowing agents. This results in insulation foams that no longer fulfill the required product specifications unless additional steps are taken to increase the thermal resistance of these insulation foams.

It is known that an infrared attenuation agent (IAA) can be used to improve an insulation foam. An effective infrared attenuation agent favors increased reflection and absorption and decreased transmission of heat radiation as much as possible. Traditionally, inorganic materials have been used as IAA to reduce the portion of heat radiation. This includes, for example, graphite, aluminum, stainless steel, cobalt, nickel, carbon black, and titanium dioxide. As an example for several documents describing IAA's, US 7,605,188 can be cited.

In addition, nucleating agents and clarifiers are commonly used in industrial practice in combination with crystallizable thermoplastic polymers to reduce processing cycle times or to impart improved physico-chemical characteristics, such as various optical, surface and mechanical properties, as well as to reduce mold shrinkage.

JP H02 284,934 A discloses the production of foamed styrene board using a combination of fluorinated aliphatic hydrocarbons and other halogenated aliphatic hydrocarbons and a selected sorbitol. JP 2009 242808 A discloses foam molded articles containing a thermoplastic resin and a compound which undergoes a solgel transition. CN 102 816 363 A discloses high resilience flame-retardant foamed polyethylene material. JP 2004 269769 A discloses a thermoplastic resin composition for foaming comprising a sorbitol compound.

The problem of the present invention is to provide a polymeric foam with outstanding insulation and mechanical properties, as well as a composition for the preparation of such a polymeric foam, in an essentially amorphous polymer.

The problem is solved by a composition comprising an at least essentially amorphous polymer and a nucleating agent of formula (1).

The subject of the present invention, as defined by the appending claims, is a composition for the preparation of a polymeric foam, and in particular a masterbatch composition, comprising
(i) an at least essentially amorphous polymer resin comprising at least 80 % by weight of an at least essentially amorphous polymer selected from the group consisting of polystyrene (PS), polystyrene copolymers, and mixtures or blends thereof, wherein the at least essentially amorphous polymer has a portion of 5 wt.-% or less of a crystalline region and at least 95 wt.-% of an amorphous region; and
(ii) a nucleating agent of formula (1) 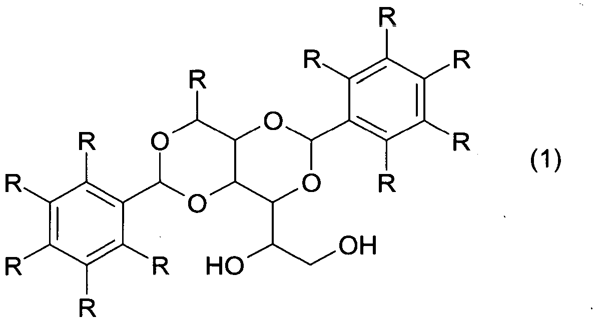 wherein
   each R independently is selected from the group consisting of hydrogen, C₁-C₁₀-alkyl, C₅-C₁₀-cycloalkyl, phenyl, phenyl substituted with 1, 2, 3 or 4 C₁-C₄-alkyl groups, and C₁-C₄-alkylene-phenyl.

Preferably, each R independently is selected from the group consisting of hydrogen, C₁-C₄-alkyl, C₅-C₆-cycloalkyl, phenyl, phenyl substituted with 1 or 2 C₁-C₃-alkyl groups, and benzyl.

More preferably, each R independently is selected from the group consisting of hydrogen, methyl, ethyl, propyl, iso-propyl, tert-butyl, 3-pentyl, neopentyl, iso-pentyl, phenyl, 4-methylphenyl, 3,4-dimethylphenyl, 3,5-dimethylphenyl, cyclopentyl, cyclohexyl, and 1-adamantyl.

A preferred nucleating agent is selected from the group consisting of 1,3:2,4-bis-(benzylidene)-sorbitol derivates of formula (I), (II) and (III) and mixtures thereof, 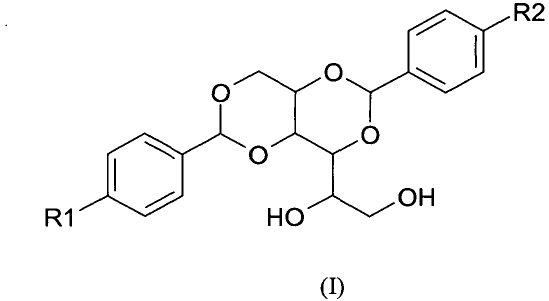 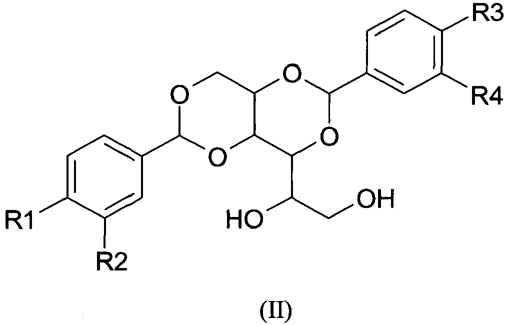 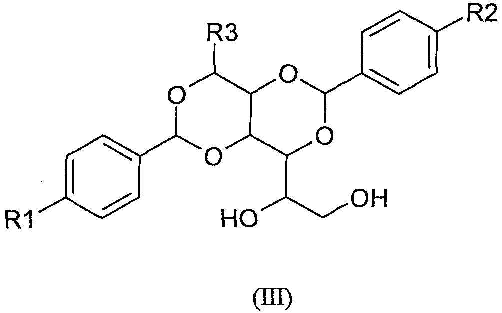 wherein
- R¹, R² , R³ and R⁴: are independently of one another selected from the group consisting of hydrogen, C₁-C₁₀-alkyl, C₅-C₁₀-cycloalkyl, phenyl, phenyl substituted with 1, 2, 3 or 4 C₁-C₄-alkyl groups, C₁-C₄-alkylene-phenyl.

More preferably,
- R¹, R², R³ and R⁴: are independently of one another selected from the group consisting of hydrogen, C₁-C₄-alkyl, C₅-C₆-cycloalkyl, phenyl, phenyl substituted with 1 or 2 C₁-C₃-alkyl groups, and benzyl.

Most preferably,
- R¹, R² , R³ and R⁴: are independently of one another selected from the group consisting of hydrogen, methyl, ethyl and propyl.

Even more preferably, R¹, R², R³ and R⁴ are each methyl or propyl.

In a particularly preferred embodiment, 1,3:2,4-bis-O-(4-methylbenzylidene)-D-sorbitol (IV), 1,3:2,4-bis-(3,4-Dimethylbenzylidene)-sorbitol (V) and 1,3:2,4-bis-(4-propylbenzylidene)-propyl sorbitol (VI) are used as the nucleating agent.

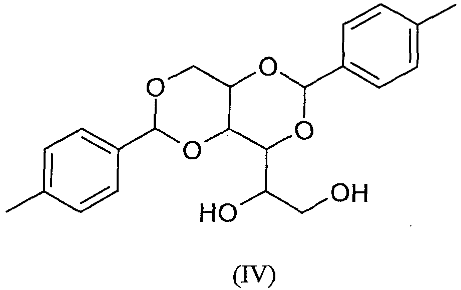

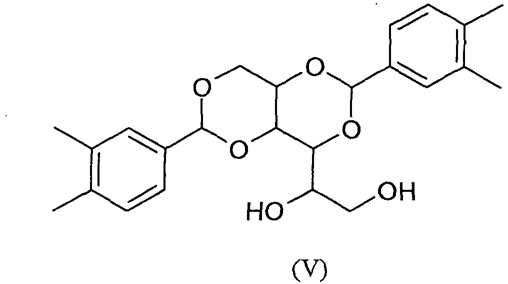

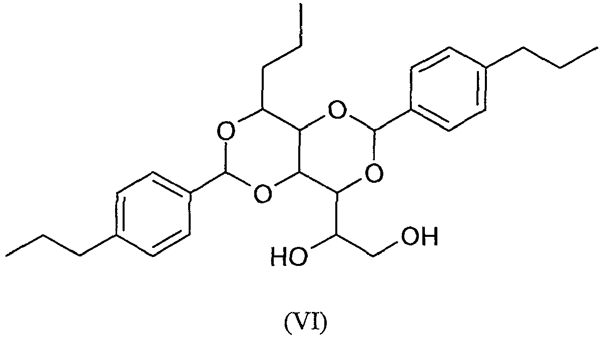

Polystyrene copolymers in the sense of the present invention are high impact polystyrene (HIPS), styrene-butadiene copolymers (SBR), styrene-butadiene-styrene copolymers (SBS), styrene-isoprene-styrene copolymers (SIS), poly(styrene-ethylene/butylene-styrene) (SEBS), styrene-ethylene/propylenestyrene copolymers (SEPS), acrylonitrile-butadiene-styrene copolymers (ABS), styrene-acrylonitrile copolymers (SAN), and acrylonitrile-styrene-acrylate copolymers (ASA).

Surprisingly, it has been found that a composition comprising at least 80 % by weight of an at least essentially amorphous polymer resin and at least one nucleating agent of formula (1) will afford a polymeric foam with significantly smaller cell sizes and with improved insulation properties. It seems that the nucleating agents of the present invention have a beneficial influence on the reduction of thermal conductivity, the foam structure, and the cell structure of the polymeric foam, the surface quality, as well as on the mechanical properties of the polymeric foam.

Also, the cell size in the polymeric foam obtainable from the composition according to the present invention is significantly reduced in comparison with conventional polymeric foams. Due to the small cell size, the polymeric foam according to the present invention has significantly better mechanical properties, such as compressive strength, modulus, or creep resistance.

Also, the polymeric foam obtainable from the composition according to the present invention has a remarkably reduced thermal conductivity (about 3 to 10 % reduction compared to standard foam) and as a consequence, the insulating effect provided by the polymeric foam is remarkably increased.

In polymer chemistry, the term "amorphous" means that there is no melting point and only a glass transition point measurable by DSC. As used herein, "at least essentially amorphous polymer" means that a portion of 5 wt.-% or less of the polymer has a crystalline region and that a portion of at least 95 wt.-% of the polymer has an amorphous region.

The "at least essentially amorphous polymer resin" used in the composition of the present invention comprises at least 80 % by weight of such an at least essentially amorphous polymer and may additionally comprise 0 to 20 % by weight of a semicrystalline or crystalline polymer blended or mixed thereto. Preferably, the "at least essentially amorphous polymer resin" comprises at least 90 % by weight of said at least essentially amorphous polymer, more preferably at least 95 % by weight of said at least essentially amorphous polymer, and most preferably 100 % by weight of said at least essentially amorphous polymer.

The synthesis of benzylidene-sorbitols is well-known in the art and e.g. described in US4371645.

In a preferred embodiment, the composition of the present invention comprises polystyrene (PS). Polystyrene is a synthetic aromatic polymer made from the monomer styrene, a liquid petrochemical. It is a very inexpensive resin per unit weight. Polystyrene is one of the most widely used plastics, the scale of its production being several billion kilograms per year.

Polystyrene foams tend to be good thermal insulators and are therefore often used as building insulation materials, such as in insulating concrete forms and structural insulated panel building systems. They are also used for non-weight-bearing architectural structures (such as ornamental pillars). PS foams also exhibit good damping properties, and are therefore widely used in packaging. Extruded closed-cell polystyrene foam is sold under the trademark ®Styrofoam by Dow Chemical Company, for instance.

Preferably, the polymer resin in the composition of the present invention comprises at least 80 % by weight, more preferably at least 90% by weight, most preferably at least 95 % by weight, of at least essentially amorphous polystyrene. An essentially amorphous polystyrene homopolymer is particularly preferred.

Essentially amorphous polystyrene has preferably a melt flow index (MFI) of 0.5 to 50 (5 kg/200 °C), more preferably an MFI of 1 to 25 (5 kg/200 °C), and most preferably an MFI of 3 to 11 (5 kg/200 °C). The MFI is determined e.g. according to ISO 1133.

Preferably, the essentially amorphous polystyrene has an average molecular weight of 30 to 500 kDa, more preferably of 100 to 400 kDa, and most preferably of 150 to 300 kDa.

The composition of the present invention may also comprise a mixture of a polystyrene having an average molecular weight of 30 to 500 kDa (preferably 80 to 99 % by weight) and a polystyrene having an average molecular weight of more than 1'000 kDa (preferably 1 to 20 % by weight). Such a mixture allows for producing thicker polymeric foams with a good quality. The production of such a polymeric foam is described in EP 1 031 600, for instance.

According to another preferred embodiment, the composition comprises ultra high molecular weight polystyrene, which preferably has an average molecular weight of 1'200 to 3'500 kDa.

The composition of the present invention may also comprise a recycled polystyrene and/or polystyrene copolymer. Thus, the at least essentially amorphous polymer resin may comprise up to 100 % of a recycled polystyrene and/or polystyrene copolymer, preferably 0 to 20 %, more preferably 5 to 20 % by weight of the total polymer resin content.

Preferably, at least part of the at least essentially amorphous polystyrene is recycled polystyrene. The recycled polystyrene may be, for example, post-consumer recycled polystyrene or post-production recycled polystyrene.

The composition of the present invention may also contain further additives as hereinafter described, alone or in combination. In a preferred embodiment, the composition of the present invention additionally comprises an inorganic nucleating agent. Such an inorganic nucleating agent is preferably selected from the group consisting of talc, e.g. nano-talc, clay, e.g. nano-clay, Halloysite clay, or Montmorillite clay, fumed silica, calcium carbonate, e.g. nano-calcium carbonate, hollow glass spheres, zeolites, magnesium-based fibers (such as Hyperform® HPR 803i from Milliken, which has an average fiber length of 25 micrometer and an aspect ratio of 50:1), and mixtures thereof. Talc has preferably an average particle size x50 of smaller than 10 µm and x98 of smaller than 30 µm, preferably an average particle size x50 of smaller than 7 µm and x98 of smaller than 21 µm, more preferably an average particle size x50 of smaller than 2 µm and x98 of smaller than 6 µm. X50 is defined as the particle diameter where half of the mass fraction of the particles is smaller and half of the mass fraction of the particles is larger than the particle diameter x50. In analogy, x98 is defined as the particle diameter where 98 % of the mass fraction of the particles is smaller and 2 % of the mass fraction of the particles is larger than the particle diameter x98.

Preferred inorganic nucleating agents are magnesium-based fibers - in particular Hyperform® HPR 803i - and talc with an average particle size x50 of smaller than 2 µm and x98 of smaller than 6 µm.

In a preferred embodiment, the composition of the present invention further comprises a blowing agent. Suitable blowing agents include non-hydrocarbon blowing agents, organic blowing agents, chemical blowing agents, and combinations thereof. A possible combination of blowing agents is, for example, a non-hydrocarbon and a chemical blowing agent, or an organic and a chemical blowing agent, or a non-hydrocarbon, an organic, and a chemical blowing agent.

Suitable non-hydrocarbon blowing agents include carbon dioxide, nitrogen, argon, water, air, nitrous oxide, helium, and combinations thereof. Most preferably, the non-hydrocarbon blowing agent is carbon dioxide.

Suitable organic blowing agents include aliphatic hydrocarbons having 1 - 9 carbon atoms, aliphatic alcohols having 1 - 3 carbon atoms, aliphatic ketones having 1 - 3 carbon atoms, aliphatic esters having 1-3 carbon atoms, aliphatic ethers having 1 - 4 carbon atoms, fully and partially halogenated aliphatic hydrocarbons having 1 - 4 carbon atoms, and combinations thereof. Preferred aliphatic hydrocarbons include methane, ethane, propane, n-butane, isobutane, n-pentane, isopentane, cyclopentane, neopentane, and petroleum ether. Preferred aliphatic alcohols include methanol, ethanol, n-propanol, and isopropanol. Preferred aliphatic ketones include acetone. Preferred aliphatic esters include methyl formate. Preferred aliphatic ethers include diethyl ether and dimethyl ether. Preferred fully and partially halogenated aliphatic hydrocarbons include fluorocarbons, chlorocarbons, and chlorofluorocarbons. Preferred chlorofluorocarbons and fluorocarbons are 1,1,1,4,4,4-hexafluoro-2-butene, 1,1-dichloro-1-fluoro-ethane, 2,2-dichloro-1,1,1-trifluoroethane, 1-chloro-1,2-difluoro-ethane (HCFC-142a), 1-chloro-1,1-difluoroethane (HCFC-142b), 1,1,1,2-tetrafluoroethane, 1,1,1,3,3-pentafluoropropane, 1,1,1,3,3-pentafluorobutane, 2-chloropropane, dichlorodifluoromethane (CFC-12), 1,2-dichloro-1,1,2,2-tetrafluoroethane, 1-chloro-1,2-difluoro-ethane, trichlorotrifluoroethane and/or trichloromono-fluoromethane (CFC-11), as well as mixtures of 1-chloro-1,2-difluoroethane (HCFC-142a) and 1-chloro-1,1-difluoroethane (HCFC-142b), 1,3,3,3-tetrafluoropropene (HFO-1234ze), 1,1-difluoroethane (HFC-152a), 1,1,1,2-tetrafluorethane (HFC-134a) and chlorodifluoromethane (R22). The aliphatic hydrocarbons or the fully and partially halogenated aliphatic hydrocarbons can also be encapsulated in microspheres (e.g. available from Akzo Nobel as Expancel®).

Preferred organic blowing agents are n-butane, iso-butane, ethanol, isopropanol, dimethyl ether, and mixtures thereof.

Preferred addition ratios are 0 to 10 wt.-%, more preferably 0.1 to 5 wt.-%, most preferably 0.5 to 4 wt.-%, of the blowing agent based on the total weight of the composition.

Preferred are mixtures of non-hydrocarbon and organic blowing agents in a ratio of 4:1 to 1:4 by weight, preferably 3:1 to 1:1, more preferably 2:1 to 1:1. Preferred mixtures contain carbon dioxide as non-hydrocarbon blowing agent and ethanol, isopropanol, dimethyl ether or mixtures thereof as organic blowing agent.

Suitable chemical blowing agents include azocarbonate-based and hydrazide-based compounds, such as azodicarbonamide, azodiisobutyronitrile, benzenesulphonyl hydrazide, 4,4'-oxy-bis-(benzenesulfonyl semicarbazide), organic acids and their derivatives, alkali metal carbonates, alkali metal bicarbonates, and mixtures thereof.

Preferred organic acids and acid derivatives include oxalic acid and oxalic acid derivatives, succinic acid and succinic acid derivatives, adipic acid and adipic acid derivatives, phthalic acid and phthalic acid derivatives, and citric acid and citric acid derivatives. More preferred are citric acid, citric acid salts, and citric acid esters, and mixtures thereof. Preferred citric acid esters are those of higher alcohols, such as stearyl or lauryl citrate, and both mono- and diesters of citric acid with lower alcohols having 1-8 carbon atoms. Suitable lower alcohols from which these citric acid esters can be formed are, for example: Methanol, ethanol, propanol, isopropanol, n-butanol, iso-butanol, sec-butanol, tert-butanol, n-pentanol, n-pentan-2-ol, n-pentan-3-ol, n-hexan-3-ol and isomeric hexanols, n-heptan-1-ol, n-heptan-2-ol, n-heptan-3-ol, n-heptan-4-ol and isomeric heptanols, n-octan-1-ol, n-octan-2-ol, n-octan-3-ol, n-octan-4-ol and isomeric octanols, cyclopentanol, and cyclohexanol. Furthermore, diols or polyols with 1-8 carbon atoms may be used, such as ethylene glycol, glycerol, pentaerythritol or lower polyethylene glycols, for example diethylene glycol, triethylene glycol or tetraethylene glycol. The mono- or diesters with monohydric alcohols having 1-6 carbon atoms are preferred and the mono- or diesters with monohydric alcohols having 1-4 carbon atoms are most preferred. The monoesters, such as monomethyl citrate, monoethyl citrate, monopropyl citrate, monoisopropyl citrate, mono-n-butyl citrate, and mono-tert-butyl citrate are particularly preferred.

Further preferred chemical blowing agents are alkali or earth alkali metal carbonates, alkali or earth alkali metal bicarbonates, such as calcium carbonate, magnesium carbonate, calcium bicarbonate, magnesium bicarbonate, ammonium bicarbonate, sodium carbonate, potassium carbonates. More preferred are calcium bicarbonate, sodium bicarbonate, and mixtures thereof.

Most preferably, the composition of the present invention comprises a blowing agent selected from the group consisting of CO₂, n-butane, iso-butane, ethanol, isopropanol, dimethyl ether, citric acid, sodium bicarbonate, and mixtures thereof.

Preferably, the composition of the present invention additionally comprises a carbon based IR absorber or a non-carbon based IR absorber to further improve the thermal properties of the foam. Possible carbon based IR absorbers are, for example, carbon black, activated carbon, graphite, carbon nanotubes, graphene, thermally reduced oxidized graphite, or graphene oxide. Especially preferred are graphite and graphene, in particular graphite. Preferably, the composition comprises the carbon based or non-carbon based IR absorber in an amount of 0 to 4 % by weight, more preferably 0.1 to 3 % by weight, relative to the total weight of the composition.

However, it is also possible to obtain excellent thermal insulation properties if the composition of the present invention comprises no carbon based IR absorbers. Such foams are perfectly white and no grey or silver shade can be observed. This is particularly advantageous because it allows for producing final articles in bright and very clean colors, e.g. bright orange, green or pink.

In addition, the composition of the present invention may further comprise a compound with flame retardant characteristics, said flame retardant preferably being selected from the group consisting of hexabromocyclododecane (HBCD), brominated polymeric flame retardants, preferably brominated polystyrene-polybutadiene block copolymers with 50 to 80 wt.-% Br content (e.g. Emerald InnovationTM 3000 from Chemtura), aluminum trihydroxide, magnesium hydroxide, antimony trioxide, zinc borate, tetrabromobisphenol A, phenoxy-terminated tetrabromobisphenol A carbonate oligomer, tert-butyl cumyl peroxide, decabromodiphenyl ethane, tetrabromobisphenol A bis(2,3-dibromopropyl ether), pentabromodiphenyl oxide, octabromodiphenyl oxide, poly(dibromostyrene), tribromophenyl allyl ether, poly(pentabromobenzyl) acrylate, and synergists, such as 2,3-dimethyl-2,3-diphenyl butane and dicumyl peroxide. Preferred flame retardants are HBCD and brominated polystyrene-polybutadiene block copolymers with 50 to 80 wt.-% Br content.

In addition, the composition of the present invention may further comprise a cell stabilizer, such as erucamide, glycerol monostearate or glycerol tristearate.

In addition, the composition of the present invention may further comprise a plasticizer. Suitable plasticizers include acrylates, fully acetylated glycerol monoester on 12-hydroxystearic acid, fully acetylated glycerol monostearate, and mixtures thereof, as well as lower molecular weight homopolymers and copolymers of acrylates. Preferred are copolymers of butyl acrylate with acrylic acid and its salts, amides and esters, with methacrylates, acrylonitrile, maleic acid esters, vinyl acetate, vinyl chloride, vinylidene chloride, styrene, butadiene, unsaturated polyesters, and drying oils. More preferred are copolymers of butyl acrylate with styrene, especially those with molecular weights lower than 80 kDa, even more preferred with molecular weights lower than 5 kDa.

In addition, the composition of the present invention may further comprise an antidripping agent, which is preferably a fluorocarbon powder, in particular polytetrafluoroethylene (PTFE).

The composition of the present invention may further comprise customary additives in a concentration range that does not adversely affect the beneficial effect of the invention, e.g. 0.0001 to 15 % by weight, preferably 0.01 to 10 % by weight, especially 0.1 to 5 % by weight, based on the total weight of the composition. Suitable customary additives include colorants, pigments, dyes, stabilizers, antioxidants, antibacterial agents, thermostabilizers, light stabilizers, neutralizers, antistatic agents, antiblocking agents, optical brighteners, heavy metal inactivation agents, hydrophobic agents, peroxides, water scavengers, acid scavengers, hydrotalcites, elastomers, impact modifiers, processing aids and the like, and also mixtures thereof.

The composition of the present invention is preferably selected from the group consisting of a masterbatch composition and a final composition.

A "masterbatch composition", as used throughout this application, is a concentrate comprising the full amount of the active agent, e.g. nucleating agent, together with a reduced amount of the at least essentially amorphous polymer or with a suitable carrier. A masterbatch composition is intended to be added to more of the at least essentially amorphous polymer resin.

The composition of the present invention may comprise the nucleating agent of formula (1) in a relative amount of 0.001 to 50 % by weight of the total weight of the composition.

If the composition is a "final composition", it preferably comprises 0.01 to 0.5 % by weight of the nucleating agent of formula (1). It is also possible to use higher concentrations of the nucleating agent, however no or only little advantage has been observed.

If the composition is a masterbatch composition, it preferably comprises 0.1 to 50 % by weight, preferably 0.2 to 30 % by weight, more preferably 0.3 to 20 % by weight, of the nucleating agent of formula (1). Such a concentrate will generally be added to (the rest of) the polymer prior to molding.

A "final composition" as used throughout this application, is a polymer composition, which is ready for the preparation of the polymeric foam. Thus, the final composition comprises all components and additives of the polymeric foam to be prepared therefrom, but not necessarily the gas forming the pores in the foam. In particular, the final composition comprises both the at least essentially amorphous polymer and the nucleating agent in the final amounts that will also be present in the polymeric foam.

The final composition of the present invention comprises at least 80 % by weight of the at least essentially amorphous polymer resin, preferably at least 90 %, and more preferably at least 95 %, while the rest adding to 100 % by weight are the nucleating agents and the further optional components as specified before.

The preferred polymer resins and preferred nucleating agents are the same for the final composition and the polymeric foam article prepared therefrom as mentioned above for the composition in general, as well as the possible additives.

The composition of the present invention, in particular a masterbatch composition, can be prepared by mixing together the respective components. The mixing of the components can occur in one step or in a plurality of steps. As mixing apparatuses for physical mixing, it is possible to use the mixing apparatuses customary in the plastics industry, preferably an apparatus selected from the group consisting of extruders, kneaders, presses, injection-molding machines, and blade mixers. Mixing preferably occurs continuously or batchwise, particularly preferably continuously. Mixing is preferably carried out at a temperature of from 80 to 330 °C, more preferably of from 130 to 300 °C, even more preferably of from 180 to 295 °C, especially of from 200 to 290 °C.

In another embodiment of the invention the components are added at room temperature to a liquid Masterbatch carrier, typically an oil, and is mixed with this carrier.

The mixing time is preferably of from 5 s to 10 h. The mixing time in the case of continuous mixing is preferably from 5 s to 1 h, more preferably from 10 s to 15 min. The mixing time in the case of batch wise mixing is preferably from 1 min to 10 h, more preferably from 2 min to 8 h, in particular from 2 min to 5 h, especially from 2 min to 1 h, particularly preferably from 2 to 15 min.

In a further aspect, the present invention also refers to a foamed polymeric article comprising
(i) 80 % to 98.99 % by weight of an at least essentially amorphous polymer,
(ii) 0.01 % to 0.5 % by weight of a nucleating agent of formula (1),
(iii) 1 to 10 % by weight of a blowing agent including carbon dioxide, nitrogen, argon, water, air, nitrous oxide, helium, and combinations thereof and/or organic blowing agent and
(iv) 0 to 18.99 % by weight of further additives as specified above.

The foamed polymeric article of the present invention is prepared from the composition of the present invention. In particular, all the preferred embodiments described above for the composition also apply to the foamed polymeric article (= polymeric foam).

Preferably, said polymeric foam has a closed cell content of more than 92 %, preferably more than 95% and more preferably more than 97 %, as determined by gas pycnometry (DIN EN ISO 4590). Such a high closed cell content results in a very smooth surface as well as in a very good resistance against water ingress.

The cells in the polymeric foam according to the present invention preferably have an average cell size of less than 100 µm, preferably of less than 80 µm, more preferably of less than 60 µm, even more preferably of less than 50 µm, and most preferably of less than 40 µm. The average cell size is generally derived from optical microscope pictures, by measuring the diameters of the foam cells and calculating their average diameter.

The polymeric foam of the present invention, in particular if it comprises polystyrene, has a reduced density of more than 90 % by weight, preferably more than 94 %, more preferably more than 95 % by weight, even more preferably more than 96.5 % and most preferably more than 97 % by weight, all compared to compact (non-foamed) polystyrene.

Preferably, such a polymeric foam has a density of 10 - 65 kg/m³, more preferably of 15 - 55 kg/m³. Preferably, the polymeric foam further has a cross sectional area of at least (20 x 1.2) cm², preferably of at least (22.5 x 1.5) cm², more preferably of at least (25 x 1.8) cm² in a continuous production process. Even more preferred, a board prepared from the polystyrene foam of the present invention passes the B2, even more preferably the B1 flame retardant test according to DIN 4102.

The polymeric foam of the invention can be prepared by a method comprising the steps of
(a) adding a nucleating agent of formula (1) as described above
   and optionally an additive as specified above, preferably a chemical blowing agent, flame retardant, inorganic nucleating agent, plasticizer, and/or colorant
   to a polymer resin as specified above, preferably in pelletized form, to obtain a polymer composition;
(b) melting the polymer composition obtained in step (a) to obtain a polymer melt; and
(c) extruding the polymer melt of step (b) in an extruder in the presence of a blowing agent;
(d) cooling the extruded polymer melt and forming the polymeric foam.

Steps (a) to (d) can be carried out in an extruder, such as a single screw extruder, a twin screw extruder or a Farrel continuous mixer. During said steps, the polymer resin is expediently heated to above the melting temperature.

Expediently, the non-hydrocarbon and/or organic blowing agents are added into the polymer melt in the mid section of the first extruder using high pressure or HPLC pumps, respectively.

The polymeric foam can be prepared by a method comprising the steps of
(a) pre-mixing the nucleating agent of formula (1) and optionally one or more of the additives as specified above with the polymer resin, preferably in pelletized form, to obtain a polymer composition;
(b) heating the polymer composition obtained in step (a) to a temperature above the melting temperature of the polymer to obtain a polymer melt;
(c) adding a non-hydrocarbon blowing agent and/or one or more organic blowing agents to the polymer melt, expediently by using a high pressure pump;
(d) homogenizing the mixture obtained in step (c) and dissolving the non-hydrocarbon blowing agent and/or the one or more organic blowing agents in the polymer melt; and
(e) extruding the homogenized mixture and cooling it in an extruder to form the polymeric foam by a pressure drop to ambient pressure or sub-ambient pressure at the extrusion die.

Steps (a) to (d) can be carried out in a first extruder, such as a single screw extruder, a twin screw extruder or a Farrel continuous mixer. Step (e) may be performed in the same first extruder, or the homogenized mixture obtained in step (d) may be transferred to a second extruder prior to the extrusion of step (e).

In both methods, the finished articles can be produced directly at the exit of the first or second extruder using an extrusion die (XPS process).

Preferably concentrates of the nucleating agent and optionally concentrates of the additives, preferably of flame retardants, inorganic nucleating agents, cell stabilizers, plasticizers, chemical blowing agents, IR absorbers, antidripping agents, and colorants, are prepared as separate masterbatches or in one or more masterbatches using the essentially amorphous polymer as carrier. These masterbatches are mixed and homogenized and fed together with the essentially amorphous polymer into the main throat of a first extruder, preferentially a corotating single screw extruder.

The polymeric foam can be prepared by a method comprising the steps of
(a) melting and mixing the masterbatches containing the nucleating agent of formula (1) and optionally the additives, preferably the flame retardants, inorganic nucleating agents, cell stabilizers, plasticizers, chemical blowing agents, IR absorbers, anti-dripping agents, and/or colorants, with the essentially amorphous polymer, preferably in pelletized form,
(b) adding the non-hydrocarbon and/or organic blowing agents to the polymer melt, expediently using a high pressure pump,
(c) dissolving the non-hydrocarbon and/or organic blowing agents in the polymer melt
(d) cooling the polymer melt near to the polymer's melting point
(e) providing a pressure drop towards ambient or sub-ambient pressure at the extruder die to produce the foam.

Alternatively, the dissolved gas can be trapped into the polymer by rapid cooling and the polymer strand exiting a round extrusion die is cut into small pellets. In this case, the finished article is created by expanding the small pellets to a desired shape by supplying energy, such as heat, water steam, microwaves, UV light. Preferably, the pellets are expanded by water steam such that they partially fuse or stick together (EPS process).

Preferably, the polymeric foam according to the present invention is prepared by a continuous process, e.g. tandem extrusion. In this case, the polymeric foam is preferably formed by an extrusion die attached to the extruder.

The polymeric foam of the present invention may be produced by sheet extrusion, board extrusion, profile extrusion, foamed sheet extrusion, of which in a second step deep drawn articles are made (e.g. for packaging, durable goods, wall decorations, food trays or food packaging). Furthermore, foam according to the present invention may be produced by blown films, extrusion blow molding or injection molding.

The polymeric foam according to the present invention may be used in an insulation board, e.g. for building and construction, including, but not limited to, perimeter insulation, thermal insulation of flat roofs, floor insulation, exterior wall insulation, ceiling heat insulation, steep roof insulation, interior fitting, sandwich boards, pipe insulation, frost protection layers for buildings and transportation routes (e.g. it can be applied as insulation beneath highways, streets, bridges, or airport runways).
Furthermore, the polymeric foam of the present invention may be used in a decorative article, including, but not limited to, construction moldings, extruded profiles, component edge moldings, window frames, picture frames, casings, moldings, foamed stocks.
Furthermore, the polymeric foam according to the present invention may be used in a packaging material for food or electronics, for medical goods or consumer goods.
Furthermore, the polymeric foam according to the present invention may be used in automotive parts, including, but not limited to, door side parts, door handles, dashboards, interior trim parts, air intake manifolds, battery housings, engine encapsulations, air-filter housings.
Furthermore, the polymeric foam according to the present invention may be used as sound insulation.

### Test methods:

The product properties are determined by the following methods, unless indicated otherwise:
Determination of the density of the produced foamed boards is carried out in accordance with ISO 1183 (kg/m³).

The average cell size (cell diameter) is derived from optical microscope pictures, by measuring the diameter of the foam cells and calculating their average diameter.

Determination of insulation properties is carried out using a Linseis Thermal Conductivity Meter, using the Transient Hot Bridge method (DIN EN 993-14, DIN EN 993-15), reporting the thermal conductivity Lambda in mW/(m*K). Determination of the open cell content is done by gas pycnometry (DIN ISO 4590).

Substances used:

| | |
|---|---|
| Component P: (at least essentially amorphous polymer) | General purpose Polystyrene (Styrolution® PS 153F) with a Melt Volume Rate (200 °C/ 5 kg, according to ISO 1133) of 75 cm³/10 min |
| Component A1: (nucleating agent) | 1,3:2,4-bis-O-(4-methiylbenzylidene)-D-sorbitol of formula (IV) |
| Component A2: (nucleating agent) | 1,3:2,4-bis-(3,4-Dimethylbenzylidene)-sorbitol of formula (V) |
| Component A3: (nucleating agent) | 1,3:2,4-bis-(4-propylbenzylidene)-propyl sorbitol of formula (VI) |
| Component B1: (inorganic nucleating agent) | Talc with an average particle size x50 of smaller than 7 µm and x98 of smaller than 21 µm |
| Component C: (non-hydrocarbon blowing agent) | CO₂ |
| Component D1: (organic blowing agent) | Ethanol |
| Component D2: (organic blowing agent) | Isobutan |
| Component E1: (chemical blowing agent) | Equimolar mixture of citric acid and sodium bicarbonate |
| Component E2: (chemical blowing agent) | Sodium bicarbonate |
| Component F1: (flame retardant) | Stabilized hexabromocyclododecane |
| Component F2: (flame retardant) | Stabilized brominated polybutadiene block copolymer with a bromine content of 64 wt.-% and a softening point of 120 °C |
| Component H1: (plasticizer) | Copolymer of butyl acrylate with styrene with molecular weights lower than 5 kDa |
| Component H2: (plasticizer) | Mixture of fully acetylated glycerol monoester on 12-hydroxystearic acid (85 - 90 wt.-%) and fully acetylated glycerol monostearate (15 - 10 wt.-%) |

In the following examples, percentages are weight percent based on the total weight of the mixture or the article, unless indicated otherwise; parts are parts by weight; "Comp." means Comparative Example.

### Preparation of masterbatches

### MB46 (inventive nucleating agent):

2.0 parts of component A1 and 98.0 parts of component P are homogenized together on a twin-screw extruder (temperature of the extruder: 180 to 240 °C) to afford masterbatch MB46.

### MB45 (inventive nucleating agent):

5.0 parts of component A2 and 95.0 parts of component P are homogenized together on a twin-screw extruder (temperature of the extruder: 180 to 240 °C) to afford masterbatch MB45.

### MB39 (inventive nucleating agent):

2.0 parts of component A3 and 98.0 parts of component P are homogenized together on a twin-screw extruder (temperature of the extruder: 180 to 240 °C) to afford masterbatch MB39.

### MB1 (inorganic nucleating agent):

10 parts of component B1 and 90 parts of component P are homogenized together on a twin-screw extruder (temperature of the extruder: 180 to 240 °C) to afford masterbatch MB1.

### MB60 (chemical blowing agent):

15 parts of component E1 and 85 parts of component P are homogenized together on a twin-screw extruder (temperature of the extruder: 80 to 180 °C) to afford masterbatch MB60.

### MB61 (chemical blowing agent):

30 parts of component E2 and 70 parts of component P are homogenized together on a twin-screw extruder (temperature of the extruder: 80 to 180 °C) to afford masterbatch MB61.

### MB17 (flame retardant):

50 parts of component F1 and 50 parts of component P are homogenized together on a twin-screw extruder (temperature of the extruder: 180 to 240 °C) to afford masterbatch MB17.

### MB16 (flame retardant):

50 parts of component F2 and 50 parts of component P are homogenized together on a twin-screw extruder (temperature of the extruder: 180 to 240 °C) to afford masterbatch MB16.

### MB41 (plastisizer):

5 parts of component H1 and 95 parts of component P are homogenized together on a twin-screw extruder (temperature of the extruder: 180 to 240 °C) to afford masterbatch MB41.

### MB44 (plastisizer):

5 parts of component H2 and 95 parts of component P are homogenized together on a twin-screw extruder (temperature of the extruder: 180 to 240 °C) to afford masterbatch MB44.

### Preparation of the polymeric foams:

The respective masterbatches are mixed and fed together with the Polymer P into a Tandem extrusion line, consisting of a first extruder, which is a twin screw extruder with 30 mm diameter and L/D of 32 and is connected to a second extruder, which is a single screw extruder with 60 mm diameter and L/D of 30 (Berstorff Schaumtandex ZE30 / KE60). The non-hydrocarbon and/or organic blowing agents are added into the polymer melt in the mid section of the first extruder using high pressure or HPLC pumps, respectively. The pressure in the first extruder is between 130 and 200 bar. The second extruder has a temperature controlled wide extrusion die of 150 mm width which can be varied in height between 0.5 and 2 mm. The pressure at the die was 68 - 76 bar.

Using a calibrator device, foamed insulation material of a width between 200 to 400 mm and a height between 12 and 35 mm is continuously produced at output rates of 30 to 40 kg/h. This continuous foamed material (foamed board) is cut into boards of the wished length. Typical haul-off speeds of the calibrator are between 2 and 5 meter per minute.

The characteristics of the polymeric foams obtained from the above masterbatches are summarized in the following examples.

In the following examples the added amount of the non-hydrocarbon and organic blowing agents is based on the total weight of the sum of polymer P and masterbatches.

### Comparative Example 1:

A foamed board was produced using 2.0 % of MB1, 3.5 % MB17, 94.5 % Polymer P, 3.3 % component C, and 2.3 % component D1. The cell diameter of this board was 280 µm and the foamed board density was 44 kg/m3.

### Example 1:

A foamed board was produced using 10.0 % of MB39, 3.5 % MB 17, 86.5 % Polymer P, 3.3 % component C, and 2.3 % component D1. The cell diameter of this board was 40 µm and the foamed board density was 55 kg/m3.

Surprisingly, using the same amount of nucleating agents the cell size was reduced from 280 µm to 40 µm.

### Comparative Example 2:

A foamed board was produced using 6 % of MB1, 3.5 % MB 17, 90.5 % Polymer P, 3.3 % component C, and 2.3 % component D1. The cell diameter of this board was 233 µm and the foamed board density was 45 kg/m3.

### Example 2:

A foamed board was produced using 10.0 % of MB 39, 4.0 % of MB1, 3.5 % MB 17, 82.5 % Polymer P, 3.3 % component C, and 2.3 % component D1. The cell diameter of this board was 60 µm and the foamed board density was 52 kg/m3.

### Comparative Example 3:

A foamed board was produced using 6 % of MB1, 3.5 % MB 17, 90.5 % Polymer P, 4.0 % component C, and 2.3 % component D1. The cell diameter of this board was 227 µm and the foamed board density was 46 kg/m³.

### Example 3:

A foamed board was produced using 10.0 % of MB 39, 4.0 % of MB1, 3.5 % MB 17, 82.5 % Polymer P, 4.0 % component C, and 2.3 % component D1. The cell diameter of this board was 37 µm and the foamed board density was 55 kg/m³. The thermal conductivity was reduced by 3 % compared to Comparative 3.

### Example 4:

A foamed board was produced using 10.0 % of MB 46, 3.0 % of MB1, 3.5 % MB 17, 83.5 % Polymer P, 4.0 % component C, and 2.3 % component D1. The cell diameter of this board was 75 µm and the foamed board density was 50 kg/m³.

### Example 5:

A foamed board was produced using 2.0 % of MB 45, 3 % MB 1, 3.5 % MB 17, 91.5 % Polymer P, 4.0 % component C, and 2.3 % component D1. The cell diameter of this board was 85 µm and the foamed board density was 45 kg/m³. A foamed board of similar density compared to the Comparative Example 3 was produced, but the thermal conductivity was reduced by 8 % compared to Comparative Example 3.

### Comparative Example 4:

A foamed board was produced using 6 % of MB1, 3.5 % MB 17, 90.5 % Polymer P, 3.3 % component C, 2.0 % component D1 and 1.0 % component D2. The cell diameter of this board was 172 µm and the foamed board density was 43 kg/m³.

### Example 6:

A foamed board was produced using 4 % of MB 45, 3 % MB 1, 3.5 % MB 17, 89.5 % Polymer P, 3.3 % component C, 2.0 % component D1, and 1.0 % component D2. The cell diameter of this board was 76 µm and the foamed board density was 54 kg/m³. The thermal conductivity was reduced by 2% compared to Comparative 4.

## Claims

1. A composition for the preparation of a polymeric foam, and in particular a masterbatch composition, comprising
(i) an at least essentially amorphous polymer resin comprising at least 80 % by weight of an at least essentially amorphous polymer selected from the group consisting of polystyrene, polystyrene copolymers, and mixtures or blends thereof, wherein the at least essentially amorphous polymer has a portion of 5 wt.-% or less of a crystalline region and at least 95 wt.-% of an amorphous region, wherein "amorphous" means that there is no melting point and only a glass transition temperature point measurable by DSC; and
(ii) a nucleating agent of formula (1) 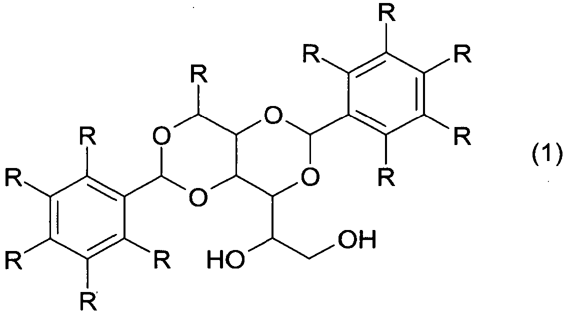 wherein
each R independently is selected from the group consisting of hydrogen, C₁-C₁₀-alkyl, C₅-C₁₀-cycloalkyl, phenyl, phenyl substituted with 1, 2, 3 or 4 C₁-C₄-alkyl groups, and C₁-C₄-alkylene-phenyl.

2. The composition according to claim 1, wherein each R independently is selected from the group consisting of hydrogen, C₁-C₄-a)ky), C₅-C₆-cycloalkyl, phenyl, phenyl substituted with 1 or 2 C₁-C₃-alkyl groups, and benzyl.

3. The composition according to claim 1, wherein each R independently is selected from the group consisting of hydrogen , methyl, ethyl, propyl, iso-propyl, tert-butyl, 3-pentyl, neopentyl, iso-pentyl, phenyl, 4-methylphenyl, 3,4-dimethylphenyl, 3,5-dimethylphenyl, cyclopentyl, cyclohexyl, and 1-adamantyl.

4. The composition according to claim 1, wherein the nucleating agent is selected from the group consisting of 1,3:2,4-bis-(benzylidene)-sorbitol derivates of formula (I), (II), (III) and mixtures thereof, 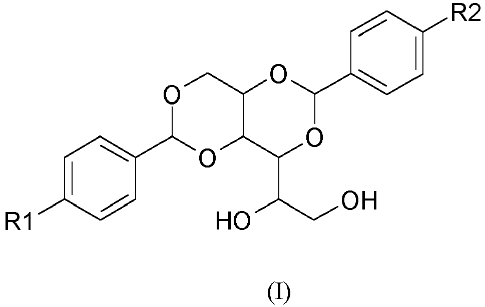 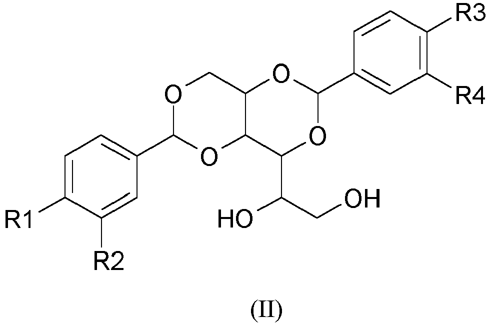 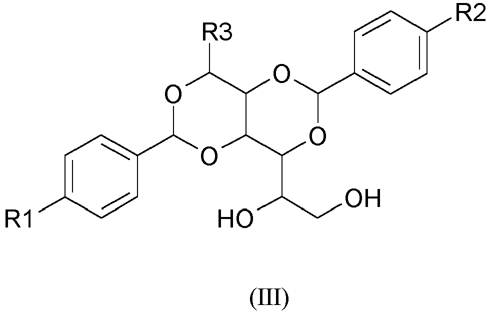 wherein
R¹, R², R³ and R⁴ are independently of one another selected from the group consisting of hydrogen, C₁-C₁₀-alkyl, C₅-C₁₀-cycloalkyl, phenyl, phenyl substituted with 1, 2, 3 or 4 C₁-C₄-alkyl groups, and C₁-C₄-alkylene-phenyl.

5. The composition according to claim 4, wherein R¹, R², R³ and R⁴ are independently of one another selected from the group consisting of hydrogen, C₁-C₄-alkyl, C₅-C₆-Cycloalkyl, phenyl, phenyl substituted with 1 or 2 C₁-C₃-alkyl groups, and benzyl.

6. The composition according to claims 4 and 5, wherein R¹, R² , R³ and R⁴ are independently of one another selected from the group consisting of hydrogen, methyl, ethyl and propyl.

7. The composition according to any of claims 4 to 6, wherein R¹, R² , R³ and R⁴ are each methyl or propyl.

8. The composition as claimed in any of claims 1 to 7, wherein the nucleating agent is selected from the group consisting of 1,3:2,4-bis-(benzylidene)-sorbitol derivates of formula (IV), (V), (VI) and mixtures thereof,

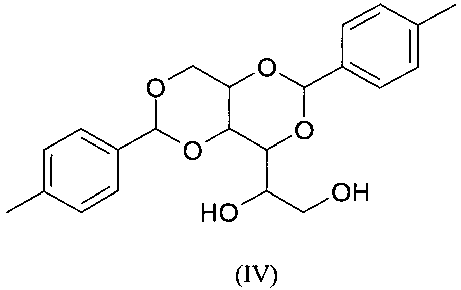

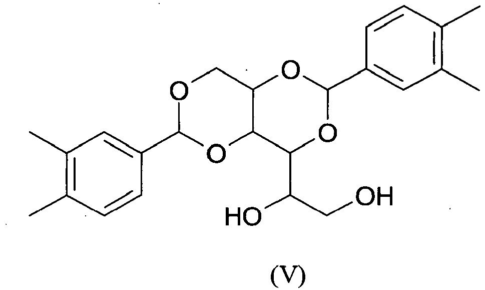

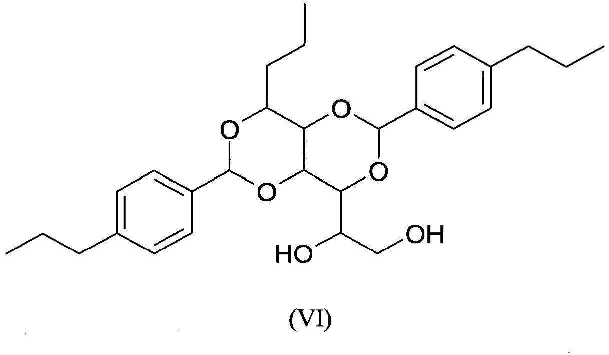

9. The composition as claimed in any of claims 1 to 8, wherein the at least essentially amorphous polymer resin is selected from the group consisting of high impact polystyrene, styrene-butadiene copolymers, styrene-butadiene-styrene copolymers, styrene-isoprene-styrene copolymers, poly(styrene-ethylene/butylene-styrene), styrene-ethylene/propylene-styrene copolymers , acrylonitrile-butadiene-styrene copolymers, styrene-acrylonitrile copolymers, and acrylonitrile-styrene-acrylate copolymers.

10. The composition as claimed in any of claims 1 to 9, **characterized in that** it further comprises a blowing agent and/or an inorganic nucleating agent.

11. The composition as claimed in any of claims 1 to 9, comprising the nucleating agent of formula (1) in a relative amount of 0.001 to 50 % by weight of the total weight of the composition.

12. A foamed polymeric article comprising (i) 80 % to 98.99 % by weight of an at least essentially amorphous polymer, (ii) 0.01 % to 0.5 % by weight of a nucleating agent of formula (1), (iii) 1 to 10 % by weight of a blowing agent including carbon dioxide, nitrogen, argon, water, air, nitrous oxide, helium, and combinations thereof and/or organic blowing agent and (iv) 0 to 18.99 % by weight of further additives, wherein (i) and (ii) are as defined in any of claims 1 to 11.

13. A method for preparing a foamed polymeric article as claimed in claim 12, comprising the steps of
(a) adding a nucleating agent of formula (1)
and optionally further additives
to an at least essentially amorphous polymer to obtain a polymer composition;
(b) melting the polymer composition obtained in step (a) to obtain a polymer melt; and
(c) extruding the polymer melt of step (b) in an extruder in the presence of a blowing agent;
(d) cooling the extruded polymer melt and forming a foamed polymeric article.

14. A method for preparing a foamed polymeric article as claimed in claim12, comprising the steps of
(a) pre-mixing the nucleating agent of formula (1) and optionally the further additives with an at least essentially amorphous polymer resin to obtain a polymer composition;
(b) heating the polymer composition obtained in step (a) to a temperature above the melting temperature of the polymer to obtain a polymer melt;
(c) adding a blowing agent including carbon dioxide, nitrogen, argon, water, air, nitrous oxide, helium, and combinations thereof and/or one or more organic blowing agents to the polymer melt;
(d) homogenizing the mixture obtained in step (c) and dissolving the blowing agent including carbon dioxide, nitrogen, argon, water, air, nitrous oxide, helium, and combinations thereof and/or the one or more organic blowing agents in the polymer melt; and
(e) extruding the homogenized mixture and cooling it in an extruder to form a foamed polymeric article by a pressure drop to ambient pressure or sub-ambient pressure at the extrusion die.

15. Use of the foamed polymeric article according to claim 12 as an insulation board, a decorative article, a packaging material for food or electronics, as sound insulation, or in automotive parts.

## Patentansprüche

1. Zusammensetzung zur Herstellung eines Polymerschaumstoffs und insbesondere einer Masterbatches-Zusammensetzung, umfassend
(i) ein zumindest im Wesentlichen amorphes Polymerharz, das mindestens 80 Gew.-% eines zumindest im Wesentlichen amorphen Polymers aus der Gruppe bestehend aus Polystyrol, Polystyrol-Copolymeren und Mischungen oder Blends davon umfasst, wobei das zumindest im Wesentlichen amorphe Polymer einen Anteil von 5 Gew.-% oder weniger eines kristallinen Bereichs und mindestens 95 Gew.-% eines amorphen Bereichs aufweist, wobei "amorph" bedeutet, dass es keinen Schmelzpunkt und nur einen durch DSC messbaren Glasübergangstemperaturpunkt gibt; und
(ii) ein Nukleierungsmittel der Formel (1) 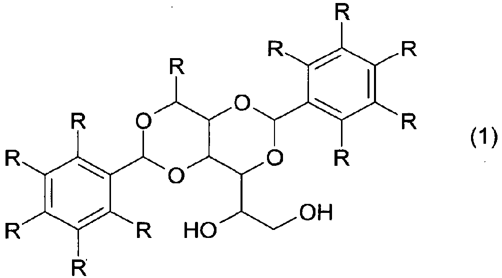 wobei
R jeweils unabhängig aus der Gruppe bestehend aus Wasserstoff, C₁-C₁₀-Alkyl, C₅-C₁₀-Cycloalkyl, Phenyl, Phenyl, das durch 1, 2, 3 oder 4 C₁-C₄-Alkylgruppen substituiert ist, und C₁-C₄-Alkylen-phenyl ausgewählt ist.

2. Zusammensetzung nach Anspruch 1, wobei R jeweils unabhängig aus der Gruppe bestehend aus Wasserstoff, C₁-C₄-Alkyl, C₅-C₆-Cycloalkyl, Phenyl, Phenyl, das durch 1 oder 2 C₁-C₃-Alkylgruppen substituiert ist, und Benzyl ausgewählt ist.

3. Zusammensetzung nach Anspruch 1, wobei R jeweils unabhängig aus der Gruppe bestehend aus Wasserstoff, Methyl, Ethyl, Propyl, Isopropyl, tert-Butyl, 3-Pentyl, Neopentyl, Isopentyl, Phenyl, 4-Methylphenyl, 3,4-Dimethylphenyl, 3,5-Dimethylphenyl, Cyclopentyl, Cyclohexyl und 1-Adamantyl ausgewählt ist.

4. Zusammensetzung nach Anspruch 1, wobei das Nukleierungsmittel aus der Gruppe bestehend aus 1,3:2,4-Bis(benzyliden)sorbitol-Derivaten der Formel (I), (II), (III) und Mischungen davon ausgewählt ist, 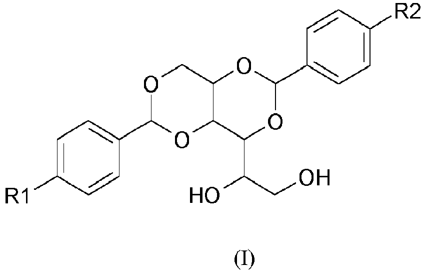 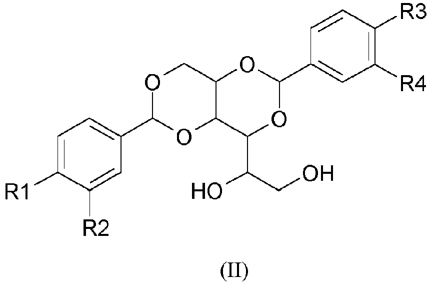 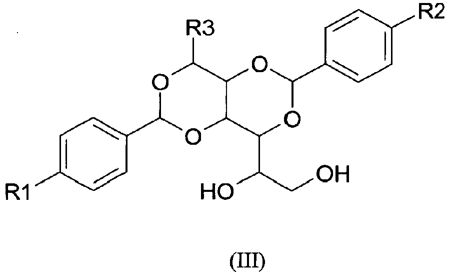 wobei
R¹, R², R³ und R⁴ unabhängig voneinander aus der Gruppe bestehend aus Wasserstoff, C₁-C₁₀-Alkyl, C₅-C₁₀-Cycloalkyl, Phenyl, Phenyl, das durch 1, 2, 3 oder 4 C₁-C₄-Alkylgruppen substituiert ist, und C₁-C₄-Alkylen-phenyl ausgewählt sind.

5. Zusammensetzung nach Anspruch 4, wobei R¹, R², R³ und R⁴ unabhängig voneinander aus der Gruppe bestehend aus Wasserstoff, C₁-C₄-Alkyl, C₅-C₆-Cycloalkyl, Phenyl, Phenyl, das durch 1 oder 2 C₁-C₃-Alkylgruppen substituiert ist, und Benzyl ausgewählt sind.

6. Zusammensetzung nach den Ansprüchen 4 und 5, wobei R¹, R², R³ und R⁴ unabhängig voneinander aus der Gruppe bestehend aus Wasserstoff, Methyl, Ethyl und Propyl ausgewählt sind.

7. Zusammensetzung nach einem der Ansprüche 4 bis 6, wobei R¹, R², R³ und R⁴ jeweils für Methyl oder Propyl stehen.

8. Zusammensetzung nach einem der Ansprüche 1 bis 7, wobei das Nukleierungsmittel aus der Gruppe bestehend aus 1,3:2,4-Bis(benzyliden)sorbitol-Derivaten der Formel (IV), (V), (VI) und Mischungen davon ausgewählt ist,

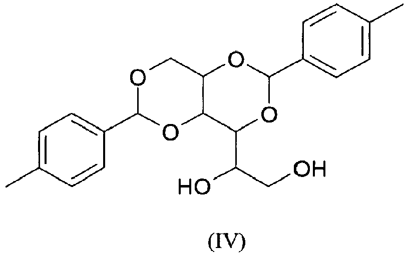

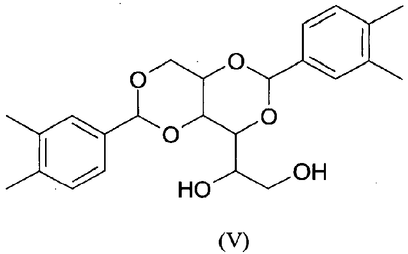

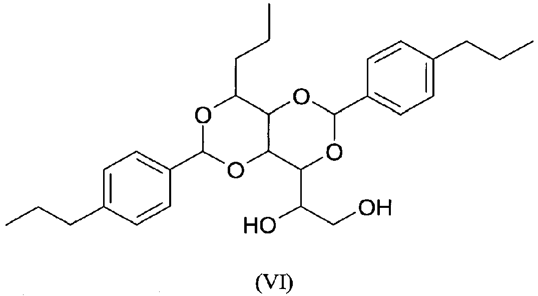

9. Zusammensetzung nach einem der Ansprüche 1 bis 8, wobei das zumindest im Wesentlichen amorphe Polymerharz aus der Gruppe bestehend aus hochschlagfestem Polystyrol, Styrol-Butadien-Copolymeren, Styrol-Butadien-Styrol-Copolymeren, Styrol-Isopren-Styrol-Copolymeren, Poly(styrol-ethylen/butylen-styrol), Styrol-Ethylen/Propylen-Styrol-Copolymeren, Acrylnitril-Butadien-Styrol-Copolymeren, Styrol-Acrylnitril-Copolymeren und Acrylnitril-Styrol-Acrylat-Copolymeren ausgewählt ist.

10. Zusammensetzung nach einem der Ansprüche 1 bis 9, **dadurch gekennzeichnet, dass** sie ferner ein Treibmittel und/oder ein anorganisches Nukleierungsmittel umfasst.

11. Zusammensetzung nach einem der Ansprüche 1 bis 9, die das Nukleierungsmittel der Formel (1) in einer relativen Menge von 0,001 bis 50 Gew.-% des Gesamtgewichts der Zusammensetzung umfasst.

12. Geschäumter Polymerartikel, umfassend (i) 80 bis 98,99 Gew.-% eines zumindest im Wesentlichen amorphen Polymers, (ii) 0,01 bis 0,5 Gew.-% eines Nukleierungsmittels der Formel (1), (iii) 1 bis 10 Gew.-% eines Treibmittels, das Kohlendioxid, Stickstoff, Argon, Wasser, Luft, Distickstoffmonoxid, Helium und Kombinationen davon enthält, und/oder ein organisches Treibmittel und (iv) 0 bis 18,99 Gew.-% weitere Additive, wobei (i) und (ii) wie in einem der Ansprüche 1 bis 11 definiert sind.

13. Verfahren zur Herstellung eines geschäumten Polymerartikels nach Anspruch 12, umfassend die Schritte
(a) Zugeben eines Nukleierungsmittels der Formel (1)
und gegebenenfalls weiterer Additive zu einem zumindest im Wesentlichen amorphen Polymers zum Erhalt einer Polymerzusammensetzung;
(b) Schmelzen der in Schritt (a) erhaltenen Polymerzusammensetzung zum Erhalt einer Polymerschmelze; und
(c) Extrudieren der Polymerschmelze aus Schritt (b) in einem Extruder in Gegenwart eines Treibmittels;
(d) Abkühlen der extrudierten Polymerschmelze und Bilden eines geschäumten Polymerartikels.

14. Verfahren zur Herstellung eines geschäumten Polymerartikels nach Anspruch 12, umfassend die Schritte
(a) Vormischen des Nukleierungsmittels der Formel (1) und gegebenenfalls der weiteren Additive mit mindestens einem zumindest im Wesentlichen amorphen Polymerharz zum Erhalt einer Polymerzusammensetzung;
(b) Erhitzen der in Schritt (a) erhaltenen Polymerzusammensetzung Temperatur oberhalb der Schmelztemperatur des Polymers zum Erhalt einer Polymerschmelze;
(c) Zugeben eines Treibmittels, das Kohlendioxid, Stickstoff, Argon, Wasser, Luft, Distickstoffmonoxid, Helium und Kombinationen davon enthält, und/oder eines oder mehrerer organischer Treibmittel zu der Polymerschmelze;
(d) Homogenisieren der in Schritt (c) erhaltenen Mischung und Lösen des Treibmittels, das Kohlendioxid, Stickstoff, Argon, Wasser, Luft, Distickstoffmonoxid, Helium und Kombinationen davon enthält, und/oder des einen oder der mehreren organischen Treibmittel in der Polymerschmelze; und
(e) Extrudieren und Abkühlen der homogenisierten Mischung in einem Extruder zur Bildung eines geschäumten Polymerartikels durch einen Druckabfall auf Umgebungsdruck oder unter Umgebungsdruck liegenden Druck an der Extrusionsdüse.

15. Verwendung des geschäumten Polymerartikels nach Anspruch 12 als Dämmplatte, dekorativer Artikel, Verpackungsmaterial für Lebensmittel oder elektronische Geräte, als Schalldämmung oder in Kraftfahrzeugteilen.

## Revendications

1. Composition pour la préparation d'une mousse polymérique, et en particulier d'une composition de mélange maître, comprenant
(i) une résine de polymère au moins essentiellement amorphe comprenant au moins 80 % en poids d'un polymère au moins essentiellement amorphe choisi dans le groupe constitué par un polystyrène, des copolymères de polystyrène, et des mélanges et des assemblages correspondants, le polymère au moins essentiellement amorphe possédant une partie de 5 % en poids ou moins d'une zone cristalline et au moins 95 % en poids d'une zone amorphe, le terme « amorphe » signifiant qu'il n'y a pas de point de fusion et seulement un point de température de transition vitreuse mesurable par DSC ; et
(ii) un agent de nucléation de formule (1) 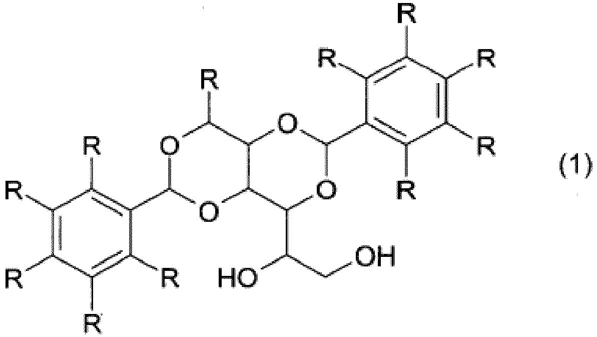 chaque R étant indépendamment choisi dans le groupe constitué par hydrogène, C₁₋₁₀-alkyle, C₅₋₁₀-cycloalkyle, phényle, phényle substitué par 1, 2, 3 ou 4 groupes C₁₋₄-alkyle, et C₁₋₄-alkylène-phényle.

2. Composition selon la revendication 1, chaque R étant indépendamment choisi dans le groupe constitué par hydrogène, C₁₋₄-alkyle, C₅₋₆-cycloalkyle, phényle, phényle substitué par 1 ou 2 groupes C₁₋₃-alkyle, et benzyle.

3. Composition selon la revendication 1, chaque R étant indépendamment choisi dans le groupe constitué par hydrogène, méthyle, éthyle, propyle, iso-propyle, tert-butyle, 3-pentyle, néopentyle, iso-pentyle, phényle, 4-méthylphényle, 3,4-diméthylphényle, 3,5-diméthylphényle, cyclopentyle, cyclohexyle, et 1-adamantyle.

4. Composition selon la revendication 1, l'agent de nucléation étant choisi dans le groupe constitué par des dérivés de 1,3:2,4-bis-(benzylidène)-sorbitol de formule (I), (II), (III) et des mélanges correspondants, 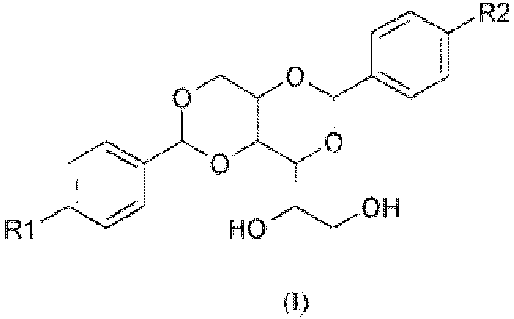 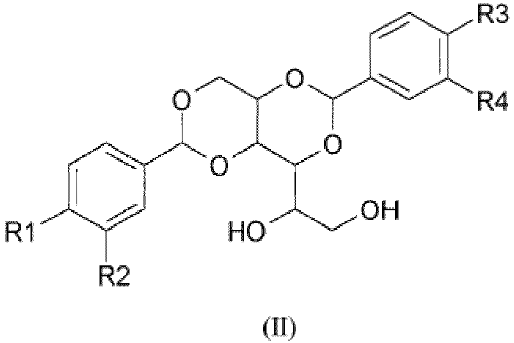 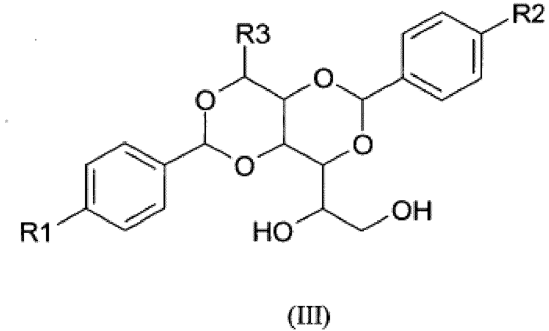 R¹, R², R³ et R⁴ étant indépendamment les uns des autres choisis dans le groupe constitué par hydrogène, C₁₋₁₀-alkyle, C₅₋₁₀-cycloalkyle, phényle, phényle substitué par 1, 2, 3 ou 4 groupes C₂₋₄-alkyle, et C₁₋₄-alkylène-phényle.

5. Composition selon la revendication 4, R¹, R², R³ et R⁴ étant indépendamment les uns des autres choisis dans le groupe constitué par hydrogène, C₁₋₄-alkyle, C₅₋₆-cycloalkyle, phényle, phényle substitué par 1 ou 2 groupes C₁₋₃-alkyle, et benzyle.

6. Composition selon la revendication 4 et 5, R¹, R², R³ et R⁴ étant indépendamment les uns des autres choisis dans le groupe constitué par hydrogène, méthyle, éthyle et propyle.

7. Composition selon l'une quelconque des revendications 4 à 6, R¹, R², R³ et R⁴ étant chacun méthyle ou propyle.

8. Composition selon l'une quelconque des revendications 1 à 7, l'agent de nucléation étant choisi dans le groupe constitué par des dérivés de 1,3:2,4-bis-(benzylidène)-sorbitol de formule (IV), (V), (VI) et des mélanges correspondants,

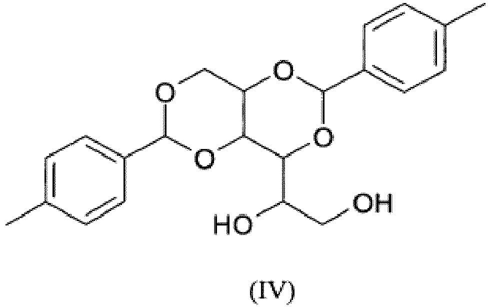

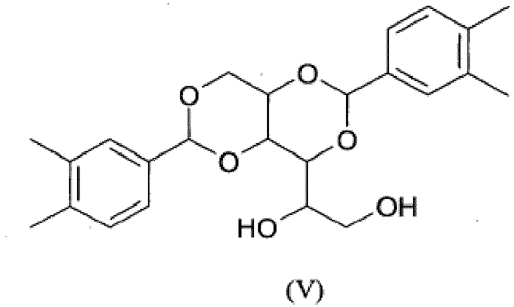

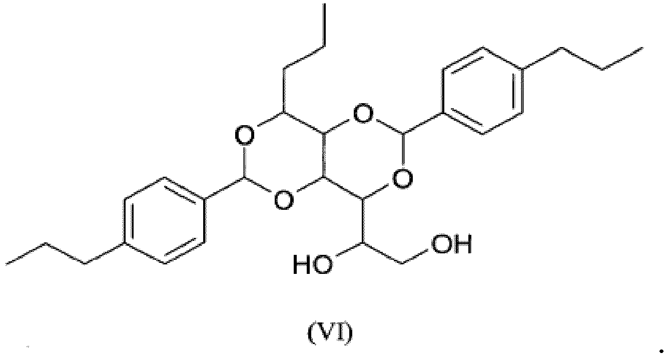

9. Composition selon l'une quelconque des revendications 1 à 8, la résine de polymère au moins essentiellement amorphe étant choisie dans le groupe constitué par un polystyrène à impact élevé, des copolymères de styrène-butadiène, des copolymères de styrène-butadiène-styrène, des copolymères de styrène-isoprène-styrène, un poly(styrène-éthylène/butylène-styrène), des copolymères de styrène-éthylène/propylène-styrène, des copolymères d'acrylonitrile-butadiène-styrène, des copolymères de styrène-acrylonitrile, et des copolymères d'acrylonitrile-styrène-acrylate.

10. Composition selon l'une quelconque des revendications 1 à 9, **caractérisée en ce qu'**elle comprend en outre un agent de gonflement et/ou un agent de nucléation inorganique.

11. Composition selon l'une quelconque des revendications 1 à 9, comprenant l'agent de nucléation de formule (1) en une quantité relative de 0,001 à 50 % en poids du poids total de la composition.

12. Article polymérique moussé comprenant (i) 80 % à 98,99 % en poids d'un polymère au moins essentiellement amorphe, (ii) 0,01 % à 0,5 % en poids d'un agent de nucléation de formule (1), (iii) 1 à 10 % en poids d'un agent de gonflement comprenant du dioxyde de carbone, de l'azote, de l'argon, de l'eau, de l'air, un oxyde nitreux, de l'hélium, et des combinaisons correspondantes et/ou un agent de gonflement organique et (iv) 0 à 18,99 % en poids d'additifs supplémentaires, (i) et (ii) étant tels que définis dans l'une quelconque des revendications 1 à 11.

13. Procédé pour la préparation d'un article polymérique moussé selon la revendication 12, comprenant les étapes de
(a) ajout d'un agent de nucléation de formule (1) et éventuellement d'additifs supplémentaires à un polymère au moins essentiellement amorphe pour obtenir une composition de polymère ;
(b) fusion de la composition de polymère obtenue dans l'étape (a) pour obtenir une masse fondue de polymère ; et
(c) extrusion de la masse fondue de polymère de l'étape (b) dans une extrudeuse en la présence d'un agent de gonflement ;
(d) refroidissement de la masse fondue de polymère extrudée et formation d'un article polymérique moussé.

14. Procédé pour la préparation d'un article polymérique moussé selon la revendication 12, comprenant les étapes de
(a) prémélange de l'agent de nucléation de formule (1) et éventuellement d'additifs supplémentaires avec une résine de polymère au moins essentiellement amorphe pour obtenir une composition de polymère ;
(b) chauffage de la composition de polymère obtenue dans l'étape (a) à une température supérieure à la température de fusion du polymère pour obtenir une masse fondue de polymère ;
(c) ajout d'un agent de gonflement comprenant du dioxyde de carbone, de l'azote, de l'argon, de l'eau, de l'air, un oxyde nitreux, de l'hélium, et des combinaisons correspondantes et/ou d'un ou plusieurs agents de gonflement organiques à la masse fondue de polymère ;
(d) homogénéisation du mélange obtenu dans l'étape (c) et dissolution de l'agent de gonflement comprenant du dioxyde de carbone, de l'azote, de l'argon, de l'eau, de l'air, un oxyde nitreux, de l'hélium, et des combinaisons correspondantes et/ou d'un ou plusieurs agents de gonflement organiques dans la masse fondue de polymère ; et
(e) extrusion du mélange homogénéisé et refroidissement de celui-ci dans une extrudeuse pour former un article polymérique moussé par une chute de pression à la pression ambiante ou à une pression inférieure à la pression ambiante au niveau de la matrice d'extrusion.

15. Utilisation de l'article polymérique moussé selon la revendication 12 en tant qu'un panneau d'isolation, un article décoratif, un matériau d'emballage pour produit alimentaire ou pour produit électronique, comme isolation sonore, ou dans des pièces automobiles.
